# EUROPEAN PATENT APPLICATION

(11) **EP 1 295 614 A1**
(43) Date of publication of application: **26.03.2003**
(21) Application number: 02256601.2
(22) Date of filing: 24.09.2002
(51) Int. Cl.: A61L 15/60

(54) **Absorbent structure and absorbent articles containing the absorbent structure**

(30) Priority: 25.09.2001 US 963050
(71) Applicant: McNEIL-PPC, INC., Skillman, NJ 08858 (US)
(72) Inventor: Nguyen, Hein, East Windsor, NJ 08520-1220 (US); Roller, Judith, North Brunswick, NJ 08902 (US); Dabi, Shmuel, Highland Park, NJ 08904 (US)
(74) Representative: Mercer, Christopher Paul

(57) **Abstract**

An absorbent structure having a mixture of fibers and superabsorbent particles, wherein the superabsorbent particles are present in the structure in an amount ranging from 2% to 60% on a weight basis, the superabsorbent particles having a residual monomer content less than 300 ppm, an absorbent capacity under low load of at least about 42 g/g and a 0.30 psi percent retention of at least about 80.5%.

## Description

### FIELD OF THE INVENTION

The present invention relates generally to an absorbent structure having improved absorbency characteristics and to sanitary absorbent articles such as sanitary napkins containing the absorbent structure.

### BACKGROUND OF THE INVENTION

Sanitary absorbent articles find wide and varied use in absorbing and trapping body fluids and maintaining body surfaces in a state of dryness and comfort. The development of materials having a high liquid absorption capacity per unit volume has allowed the required overall thickness of sanitary absorbent articles to be reduced providing products which are less obtrusive to wear. Such articles find use, for example in feminine protection devices such as sanitary absorbent napkins. The sanitary napkins generally include a top sheet which is placed nearest the body surface of the wearer, an acquisition or transfer sheet with a relatively open structure having a relatively high void volume for accepting fluid and transporting fluid to an absorbent core which serves as the main repository for liquid absorbed by the napkin, a barrier sheet which is impervious to liquid absorbed into the absorbent core and serves as a protective barrier between the absorbent core material and the wearer's clothing. The absorbent core has a high liquid absorption capacity relative to the top and transfer sheets and can be made from materials such as wood pulp, creped cellulose wadding, absorbent foams and sponges, polymeric fibers and polymeric gelling agents.

A problem with many conventional sanitary absorbent napkins is their ability to retain the absorbed liquid when subjected to mechanical loads as would be applied by the wearer in use. When subjected to such loading, liquid can leak from the absorbent core and rewet the layers above through which liquid was originally passed to the absorbent core. As the transfer and cover layers are made from materials with little absorption capacity, the liquid expelled from the absorbent core will tend to reside next to the body surface of the wearer resulting in discomfort and possible staining of the wearers garments. Typically in the prior art, this problem was addressed by utilizing materials of construction of the thin sanitary absorbent article utilizing superabsorbents having high "absorbency under load", or AUL. To achieve high AUL's the superabsorbents tend to have high crosslink density which leads to limited intrinsic potential absorbent capacity (free swell under no or very little load). This was to be the obvious choice, the other being lightly crosslinked material which would yield very high free swell capacity but does not hold up very well under compressive loads and additionally the residual monomer content tends to be high.

### SUMMARY OF THE INVENTION

In accordance with the present invention there is provided an absorbent structure comprising a mixture of fibers and superabsorbent particles, wherein the superabsorbent particles are present in the structure in an amount ranging from 2% to 60% on a weight basis, the superabsorbent particles having a residual monomer content less than 300 ppm, an absorbent capacity under low load of at least about 42 g/g and a 0.30 psi percent retention of at least about 80.5%.

### BRIEF DESCRIPTION OF THE DRAWINGS

Examples of embodiments of the present invention will now be described with reference to the drawings, in which:
Figure 1 is a top elevational view of a sanitary napkin in accordance with an embodiment of the present invention, the cover layer of the sanitary napkin being partly removed to show the absorbent system; and
Figure 2 is a side cut-away view of an embodiment of a sanitary napkin in accordance with an embodiment of the present invention.

### DESCRIPTION OF THE PREFERRED EMBODIMENTS

In an absorbent structure, such as a sanitary napkin, not every part of the product is under pressure at any one time, nor any one part of the product is under pressure all the time. So it is much more important that the superabsorbent particles possess high fluid retentive capability, i.e. under occasional pressure can retain the fluid it already absorbed while under no or little pressure. Consequently it is desirable to make practical use of the superabsorbents that have high free swell absorbency and high retentive capability even if their AUL (Absorbency Under Load) values may be very low. Suitable superabsorbents for use in this invention have both very high free swell absorbency and high retention under load even though the absorbency under load may be low. It is also desirable to utilize those superabsorbents with low residual monomer content. However, high free swell capacities tend to be associated with high residual monomer content. The present invention is directed to superabsorbents with high free swell/high retention properties with residual monomer content below 300ppm. More particularly, the superabsorbent particles of the present invention have a residual monomer less than 300ppm, an absorbency under low load greater than 42g/g, 0.3-psi percent retention greater than 80.5%, preferably greater than 74%. In addition, the superabsorbent particles preferably have a ratio of retention to absorbency under load (RAR) greater than 1.6 at 0.3 psi and greater than 5.0 at 0.7 psi.

In addition, the present invention is directed to an absorbent structure comprising a mixture of fibers and superabsorbent particles, wherein the superabsorbent particles are present in the structure in an amount ranging from 2% to 60% on a weight basis, the superabsorbent particles having a residual monomer content less than 300 ppm, an absorbent capacity under low load of at least about 42 g/g and a 0.30 psi percent retention of at least about 80.5%. The absorbent structure of the present invention is suitable for use in a variety of absorbent articles, including, but not limited to sanitary napkins, diapers, incontinence products, nursing pads and the like. For purposes of illustration, the use of the absorbent structure will be described in a sanitary napkin, however, the utilization of the absorbent structure in other absorbent articles will be readily apparent to those skilled in the art.

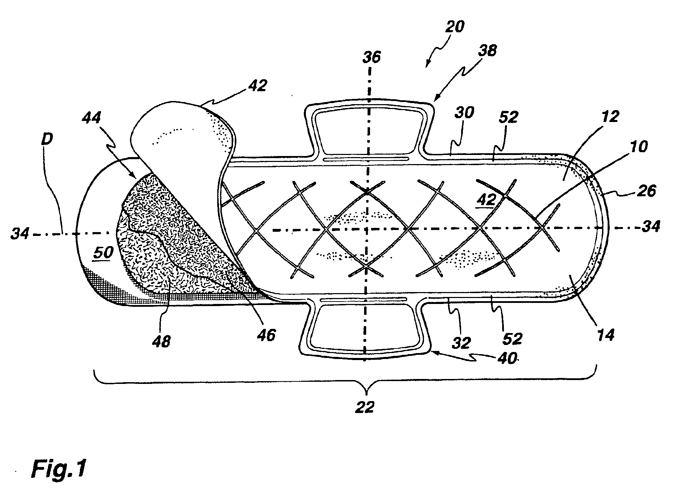

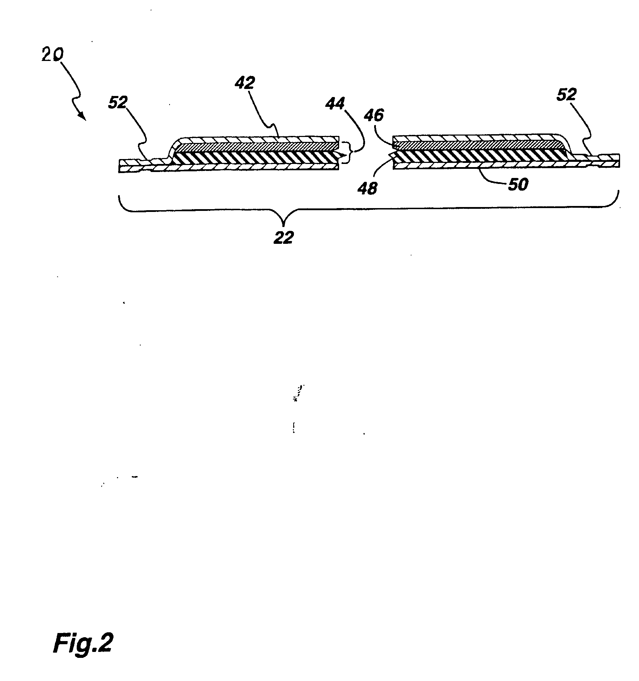

Referring to Figures 1 and 2, there is shown an embodiment of the present invention, wherein the absorbent structure is utilized in a feminine sanitary napkin 20. The sanitary napkin 20 has a main body 22 with a first transverse end 26 defining a front portion thereof and a second transverse end 28 defining a rear portion thereof and defining therebetween a length. Each of these ends is preferably arcuate. The main body also has two longitudinally extending sides, 30 and 32 on opposite side of the napkin defining therebetween a width. The sanitary napkin 20 has a longitudinal centerline 34 that is an imaginary line bisecting the sanitary napkin 20 in two substantially identical halves. The main body 22 also has an imaginary transverse line 36 perpendicular to the longitudinal centerline 34.

As depicted in Figure 2, the main body 22 is of a laminate construction and preferably comprises a fibrous fluid-permeable body facing cover layer 42, an absorbent system 44, and a fluid-impervious barrier layer 50. The absorbent system may be formed from a single layer of absorbent material or alternatively may be formed as a laminate of two or more layers of absorbent material. In a preferred embodiment, the absorbent system comprises two component layers, namely a first absorbent layer 46 (commonly known as "transfer layer") and a absorbent structure 48 (commonly known as "absorbent core"). Alternatively, a single layer, namely the absorbent structure 48, can form the absorbent system 44. Each of these layers is described in hereinbelow.

### Main Body--Cover Layer

The cover layer 42 may be formed from any flexible, liquid permeable material that is non-irritating to a user. Suitable liquid permeable materials include, but are not limited to woven fabrics, non-woven fabrics, apertured plastic films, and the like. The cover layer 42 is preferably a relatively low density, bulky, high-loft non-woven web material. The cover layer 42 may be composed of only one type of fiber, such as polyester or polypropylene or it may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Bi-component fibers may be made up of a polyester layer and a polyethylene sheath. The use of appropriate bi-component materials results in a fusible non-woven fabric. Examples of such fusible fabrics are described in U.S. Patent 4,555,446 issued November 50, 1985 to Mays. Using a fusible fabric increases the ease with which the cover layer may be mounted to the adjacent first absorbent layer and/or to the barrier layer.

The cover layer 42 preferably has a relatively high degree of wettability, although the individual fibers comprising the cover may not be particularly hydrophilic. The cover material should also contain a great number of relatively large pores. This is because the cover layer 42 is intended to take-up body fluid rapidly and transport it away from the body and the point of deposition. Advantageously, the fibers which make up the cover layer 42 should not lose their physical properties when they are wetted, in other words they should not collapse or lose their resiliency when subjected to water or body fluid. The cover layer 42 may be treated to allow fluid to pass through it readily. The cover layer 42 also functions to transfer the fluid quickly to the other layers of the absorbent system 44. Thus, the cover layer 42 is advantageously wettable, hydrophilic and porous. When composed of synthetic hydrophobic fibers such as polyester or bi-component fibers, the cover layer 42 may be treated with a surfactant to impart the desired degree of wettability. An alternative embodiment would be that the cover layer 42 is composed of an apertured film.

The cover layer 42 may be affixed, e.g., by embossing to the remainder of the absorbent system 44 by affixing the cover to the underlying layer in order to assist fluid transport from the cover to the absorbent system. Such affixation may be effected locally, at a plurality of sites or over the entire contact surface of the cover layer 42 absorbent system 44. Exemplary means of affixing the cover layer to the absorbent system 44 are adhesion and fusion.

### Main Body - Absorbent System -First Absorbent Layer

Adjacent to the cover layer 42 on its inner side and bonded to the cover layer 42 is a first absorbent layer 46 that forms part of the absorbent system 44. The first absorbent layer 46 provides the means of receiving body fluid from the cover layer 42 and holding it until an underlying absorbent structure has an opportunity to absorb the fluid, and therefore acts as a fluid transfer or acquisition layer.

The first absorbent layer 46 is, preferably, more dense than and has a larger proportion of smaller pores than the cover layer 42. These attributes allow the first absorbent layer 46 to contain body fluid and hold it away from the outer side of the cover layer 42, thereby preventing the fluid from re-wetting the cover layer 42 and its surface. However, the first absorbent layer 46 is, preferably, not so dense as to prevent the passage of the fluid through the layer 46 into the underlying absorbent structure 48.

The first absorbent layer 46 may be composed of fibrous materials, such as wood pulp, polyester, rayon, or the like, or combinations thereof. Alternatively, the first absorbent layer 46 may be composed of non-fibrous materials such as flexible foam. In a preferred embodiment, the first absorbent layer 46 is composed of fibrous materials and may include thermoplastic fibers for the purpose of stabilizing the layer and maintaining its structural integrity. The first absorbent layer 46 may be treated with surfactant on one or both sides in order to increase its wettability, although generally the first absorbent layer 46 is relatively hydrophilic and may not require treatment. The first absorbent layer 46 is preferably bonded on both sides to the adjacent layers, i.e. the cover layer 42 and an underlying absorbent structure 48.

Materials particularly suitable for use in the first absorbent layer 46 have a density in the range of about 0.04 to 0.12 g/cc, a basis weight in the range from about 30 to 110 g/m² and a thickness in the range of about 2 to 3 mm and in particular a thickness of 2.6 mm. Examples of suitable materials for the first absorbent layer are through air bonded pulp sold by Buckeye of Memphis, Tennessee, under the designation VIZORB 3008, which has a basis weight of 110 g/m² and VIZORB 3010, which has a basis weight of 90 g/m².

### Main Body -- Absorbent System―Absorbent structure

Immediately adjacent to and bonded to the first absorbent layer 46 is the absorbent structure 48. As noted above, the absorbent system preferably comprises both the first absorbent layer and the absorbent structure. In one embodiment, the first absorbent layer 46 has a width that is at least about the same as the width of the absorbent structure 48. In another embodiment, the first absorbent layer 46 has a width that exceeds the width of the absorbent structure 48.

The absorbent structure 48 contains a superabsorbent polymer (SAP). For the purposes of the present invention, the term "superabsorbent polymer" (or "SAP") refers to materials which are capable of absorbing at least about 10 times their weight in body fluids. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. Of Osaka, Japan, under the designation of SA70N. In one embodiment, the absorbent structure 48 is a blend or mixture of cellulosic fibers and superabsorbent disposed in and amongst fibers of that pulp. In a specific example, the absorbent structure 48 is a material containing from about 40 weight percent to about 98 weight percent cellulosic fibers and, more specifically from about 60 to about 80 weight percent cellulosic fibers. Such a material may contain from about 2 weight percent to about 60 weight percent SAP (superabsorbent polymers), preferably form about 10 to about 55 weight SAP, and even more preferably from about 20 to 45 weight percent SAP, and most preferably about 25 to 35 weight percent SAP.

Cellulosic fibers that can be used in the absorbent structure 48 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material.

It is an advantage of the present invention that the composite structures containing the superabsorbents described can achieve very high absorbent capacity and retention capacity. In addition, because of higher potential absorbent and retention capacities it is possible to use lower superabsorbent contents thus enhancing the mechanical properties of the composite structure. For the same reason it is possible to use lower total basis weight composite structure yielding a thinner product.

The absorbent structure 48 can be manufactured by using air-laying means. In accordance with conventional manufacturing techniques well known in the art (not shown in the drawings), cellulosic fibers (e.g., pulp) are processed using a hammer mill to individualize the fibers and may be blended with synthetic fiber, i.e.polyester fibers, acetate fibers, fusible bi-component fibers, cellulose staple fibers such as rayon, and the like and combinations thereof. The individualized fibers are conveyed into a series of forming heads and blended with SAP granules in a blending system. The blending and distribution of fibers and SAP granules can be controlled separately for each forming head. The SAP can be thoroughly and homogeneously blended throughout the material or contained only in specific strata by distributing it to selected forming chambers. Fibers (and SAP) from each forming chamber are deposited by vacuum onto a forming wire thus forming a layered absorbent web. Chemical, thermal of hydrogen bonding means, or combinations thereof, subsequently binds the web. Chemical bonding involves the application of a binder, preferably water-based latex, which is sprayed or coated onto the surfaces of the web, with the assistance of vacuum, and the web is then dried and cured. Thermal bonding is achieved by passing hot air through an air laid structure that contains bi-component or other fusible fibers. In hydrogen bonding, the web is compressed using heated calender rolls to achieve desirable density. The bonded web is wound into a roll using conventional winding equipment. The forming wire can be covered with tissue paper to reduce the loss of material. The tissue paper layer can be removed prior to bonding or incorporated into the formed material. In a possible variant, the first absorbent layer 46 can be formed integrally with the absorbent structure 48 to provide a unitized absorbent system 44. This can be achieved by providing the apparatus with an additional forming head to deposit on the absorbent structure 48, by air laying and prior to bonding, a layer of material to form the first absorbent layer 46.

Air-laid absorbents are typically produced with a low density . However, the preferred absorbent structure 48 of the present invention is calender bonded to high density and in a specific example has a density of greater than about 0.25 g/cc. Specifically, the absorbent structure 48 may have a density in the range of from about 0.25 g/cc to about 0.50 g/cc. More specifically, the density is from about 0.25 g/cc to about 0.40 g/cc and, even more specifically from about 0.25 g/cc to about 0.35 g/cc.

The absorbent structure 48 can be prepared over a wide range of basis weights. The absorbent structure 48 can have a basis weight in the range of from about 100 g/m²to about 700 g/m². In a specific example, the basis weight ranges from about 150 g/m² to about 400 g/m². Preferably the basis weight ranges from about 180 g/m² to about 350 g/m² and, more preferably, to about 200 g/m².

The first absorbent layer 46 functions to rapidly absorb and retain fluid which is then more slowly absorbed by the absorbent structure 48. The first absorbent layer having a relatively open pore structure readily absorbs and disperses liquid laterally within its bulk and readily transfers the liquid to the receiving surface of the absorbent core. In turn, the absorbent core having a relatively smaller pore structure than the first absorbent layer has good capillarity which efficiently draws liquid into its bulk from the first absorbent layer. Once the liquid has been absorbed into superabsorbent polymer, the liquid cannot be subsequently released by applying pressure. Therefore, the liquid absorbed into the superabsorbent material becomes entrapped. At the same time, the strength with which absorbent structure intakes liquid from the first absorbent layer helps to reduce the proportion of liquid held in the first absorbent layer, thereby reducing the amount of liquid that returns to the cover layer when the napkin is subjected to mechanical loading. Furthermore, the first absorbent layer has a relatively high capillarity so that any concentration of liquid in the first absorbent layer resulting from mechanical loading can be redistributed within the material to lower concentrations, again reducing the amount of liquid which can return to the cover layer.

### Main Body--Barrier Layer

Underlying the absorbent system 44 is a barrier layer 50 comprising liquid-impervious film material so as to prevent liquid that is entrapped in the absorbent system 44 from egressing the sanitary napkin and staining the wearer's undergarment. The barrier layer 50 is preferably made of polymeric film, although it may be made of liquid-impervious air-permeable material such as repellent-treated, non-woven or microporous films or foams.

The cover layer 42 and the barrier layer 50 are joined along their marginal portions so as to form an enclosure or flange seal that forms a unitary absorbent product and maintains the absorbent system 44 captive. The joint may be made by means of adhesives, heat-bonding, ultrasonic bonding, radio frequency sealing, mechanical crimping, and the like and combinations thereof. The peripheral seal line is shown in Figure 1 by the reference numeral 52.

### Flaps

Projecting laterally outward from each of the longitudinal sides 30, 32 is a flap 38, 40 (respectively). The flaps 38, 40 are in the shape of an isosceles trapezoid with the top adjoining the longitudinal side and the base at the distal end. The flaps 38 and 40 are preferably made as integral extensions of the cover layer 42 and the barrier layer 50. These integral extensions are joined to one another along their marginal seal portions by adhesives, heat-bonding, ultrasonic bonding, radio frequency sealing, mechanical crimping, and the like and combinations thereof. Most preferably, such joining is made at the same time the cover layer 42 and the barrier layer 50 are bonded to one another to enclose the absorbent system 44. Alternatively, the flaps may include absorbent material between the cover layer and the barrier layer extensions. Such absorbent material may be an extension of the first absorbent layer 46, the absorbent structure 48 or both.

### Adhesive system

Referring to Figures 2 and 3, in order to enhance the stability of the sanitary napkin, the garment facing surface of the barrier layer is provided with an undergarment attachment or positioning adhesive material 58, typically hot-melt adhesive material capable of establishing a temporary bond with the undergarment material. A suitable material is the composition designated HL-1491 XZP commercially available from H.B. Fuller Canada, Toronto, Ontario, Canada. The positioning adhesive 58 may be applied to the garment-facing surface of the barrier layer 50 in various patterns, including complete adhesive coverage, parallel longitudinal lines, a line of adhesive following the perimeter of the structure, transverse lines of adhesive or the like.

A strip of release paper 82 (shown only in Figure 3) covers the positioning adhesive 58 before the napkin is used to prevent the unwanted adherence of the napkin to itself or foreign objects. The release paper is of conventional construction (e.g. silicone coated wet-laid Kraft wood pulp) and suitable papers are available from Tekkote Corporation (Leonia, New Jersey, USA), and bear the designation FRASER 30#/61629.

### CHANNELS

In a preferred embodiment, the sanitary napkin is provided with at least one and preferably more than one channel arranged to direct liquid along the channel (or channels) for subsequent absorption into the first absorbent layer. The channels are formed by embossing and densifying one or more regions of the napkin. It has been found that the provision of channels adjacent the cover layer enables liquid to be transported rapidly over the napkin so that different regions of the first absorbent layer act effectively to absorb the liquid in parallel and contributes significantly in reducing the rewet potential. This helps to ensure that liquid is presented to a larger portion of the surface area of the absorbent structure to increase the effectiveness of the absorbent structure in drawing liquid from the first absorbent layer. Preferably, the napkin has a plurality of elongate channels formed therein, which are spaced apart from each other and configured to channel liquid laterally across the body-facing surface of the napkin or near body facing surface portion thereof, away from the region of initial deposition.

The napkin may be provided with a single channel or multiple channels, for example running along or parallel to the longitudinal axis along the length of the napkin, obliquely of the longitudinal axis, for example from one side of the napkin to the other or substantially perpendicular to the longitudinal axis. The channel(s) may have any shape which may be selected according to the particular application, for example the channels may be linear, arcuate or have a serpentine configuration or a mixture of these as well as other shapes, including a spiral and zigzag patterns.

In one embodiment, the napkin has a plurality of discrete channel formations which are spaced apart and intersect one another. An example of such an embodiment is shown in Figure 1. Referring to Figure 1, the napkin 20 is provided with a plurality of arcuate channels 10 which extend generally obliquely of the longitudinal center line 34 from one half of the napkin surface formed by the center line 34 to the other half. This design efficiently conducts liquid simultaneously along the length and across the width of the napkin. The channel formation may be formed in the cover layer and/or in the first absorbent layer. The channels may be formed advantageously by applying localized pressure to the material as for example is used in embossing. The applied pressure results in densifying the material which defines the floor of the channel rendering it less pervious to liquid and so extending the distance over which the liquid can travel before absorption. The first absorbent (transfer) layer is preferably relatively thick in comparison with the other layers of the napkin which enables relatively deep channels to be formed. Advantageously, portions of the transfer layer laterally adjacent to the channel remain relatively thick and retains their original, relatively open pore structure allowing liquid to be efficiently drawn from the channel. Advantageously, the transfer layer comprises thermoplastic fibers. The provision of thermoplastic fibers assists in the formation of a stable and permanent channel when the thermoplastic fibers and subjected to heat. When heat is applied, the thermoplastic fibers tend to fuse together to form a more rigid structure so that the original form of the channels is maintained during use and over time. Conveniently, the application of heat may be incorporated with the embossing process.

### Method of manufacture

The above-described embodiment of the sanitary napkin 20 is fabricated in a conventional manner in accordance with conventional techniques. Specifically, a laminate structure, sometimes referred to in the art as a web, is created. This laminate structure comprises an expanse of the materials from which the napkin will be created. In other words, the laminate structure comprises the following layers of material in a top-to-bottom order: an expanse of cover layer material; an expanse of first absorbent layer material; an expanse of absorbent structure material (manufactured as described above); and finally an expanse of barrier layer. Some of the materials are necessarily not continuous within the laminate structure, and where such is the case, they are positioned precisely, one with respect to another, in the relationship they will occupy in the final products. The cover layer material and the barrier layer material are then bonded together by applying pressure in the appropriate positions, and what will become the peripheral seal is created. (The seal may also be made by means of heat-bonding, ultrasonic bonding, radio frequency sealing, mechanical crimping, and the like and combinations thereof.) The sealed structure is then severed by conventional means (i.e. die-cutting, fluid-jet cutting, or by laser) from the web to create a discrete article.

As mentioned above, one or more channels may be formed adjacent the body facing surface of the napkin, and the channel(s) may be formed for example by embossing. The channel(s) may be formed by other techniques, including cutting, excavating, etching, molding and cauterizing, as well as other methods known to those skilled in the art. If embossing is used, the method may involve passing the sanitary napkin between a pair of rollers, in which one of the rollers includes projections configured to the desired embossing pattern. The projections compress and densify the material locally and may be applied to the cover layer, the absorbent system (particularly, the first absorbent layer) or a combination of the two. The degree of pressure applied during the embossing operation depending upon the type of material and its physical integrity. Finding the optimal process conditions in accordance with the specific application is within the scope of a person skilled in the art. In general, the embossing pressure should be selected to sufficiently densify the material locally to form the channels but not too high so as to sever the material. As mentioned above, the material may also be heated and this may be done conveniently by heating the embossing rollers. Ultrasonic embossing may also be used for forming the channel(s).

Advantageously, embossing helps to hold the various layers of the sanitary napkin together and reduces the likelihood of the cover layer or the barrier layer separating from the adjacent layers or coming loose when the sanitary napkin is bent. Preferably, the napkin is embossed at regular intervals over the majority and preferably the entirety of its surface.

The positioning adhesive material is then applied to the barrier layer in the appropriate positions, and release paper is applied to cover the positioning adhesive. Alternatively, the positioning adhesive, or the positioning adhesive and the release paper may be applied to the web before the individual articles are severed therefrom.

### EXAMPLES

Selected superabsorbent powders were tested for Absorbency under low load, retention capacity and Ratios of Retention to AUL (RAR) were evaluated in the in the following manner.
* The powder was pre-screened to keep only those particles which were retained on top of U.S. Standard 100-mesh screen.
* A device used in the test consists of a hollow Plexiglas cylinder with 1-in ID and a 100-mesh metal screen covering one end, the other end was open.
* 0.10 gram of powder was placed in the cylinder, deposited as evenly as possible on the screen.
* The assembly was placed on top of a multi-hole test cell which was part of a GAT (Gravimetric Absorbency Testing) system: a GF/A filter paper covered the holes providing a porous medium to conduct the test fluid. The test cell was raised so that the filter paper was 1 cm above the fluid level in the reservoir. The test fluid was 0.9% saline.
* "Free swell absorbency" is defined as absorbency under a restraining load of 0.01 psi. (for a description of the test apparatus reference se U.S. Patent 5,601,542 to Melius which is incorporated herein by reference in its entirety) A 4.4 gram weight Plexiglas puck machined to closely fit but not bind the cylinder was placed on top of the spread out powder to provide 0.01 psi pressure. The test free swell absorbency test was run for an hour to enable all of the superabsorbents to reach their equilibrium capacity. Free swell absorbency was reported as g fluid picked up / g of dry SAP.
* Retention capacity was measured on the same sample after the free swell absorbent capacity has been reached.: A weight was placed on top of the 4.4 g puck to apply the desired pressure (either 100 g for 0.3 psi or 250 g for 0.73 psi) until a new equilibrium was established, typically from 15 to 30 minutes. The amount of fluid retained by the sample was reported in g of fluid retained / g of dry SAP .
* In a test intended for AUL (absorbency under load) measurement an appropriate weight was placed on top of the plug at the beginning of the test.

Dry samples were based on a moisture content specified by the manufacturer for each material listed. Samples were not dried for the test. The superabsorbents tested were J-550, SA-60N type 2, SA-65N, SA-70N, SA-70S, 10-SH which are commercially available from Sumitomo, IM-1000A commercially available from the Sanyo Corporation, SAP powders isolated from HUGGIES® diapers which are commercially available from Kimberly-Clark and SAP powders isolated from PAMPERS® diapers which are commercially available from Procter & Gamble.

The data are listed in Table I; RAR's (Retention over AUL Ratio) are reported in the two columns on the right. Further analysis of Percent Retention is listed in Table II

**Table I:**

| Raw data and RAR (Retention /AUL Ratio) | | | | | | | | |
|---|---|---|---|---|---|---|---|---|
| | RETENTION 1 | | RETENTION 2 | | AUL1 | AUL2 | RETAINED/AUL (RAR) | |
| | 0psi ----> 0.3psi | | 0psi----> 0.7psi | | 0.3 psi | 0.7psi | 0.3 psi 0.7psi | |
| SAP in Huggies ® PA110607F | 36.5 | 30.2 | 37.1 | 27.6 | 24.3 | 13.4 | 1.24 | 2.06 |
| | | | | | | | | |
| SAP in Pampers ® 1115UG11912002 | 33.8 | 27.5 | 34.8 | 25.7 | 23.7 | 19.9 | 1.16 | 1.29 |
| | | | | | | | | |
| J-550 | 47.6 | 33.9 | 48.7 | 30.3 | 22.1 | 8.1 | 1.59 | 3.90 |
| | 47.0 | 34.6 | 48.7 | 31.3 | 21.1 | 7.7 | | |
| | | | | | | | | |
| 10-SHP | 41.1 | 29.7 | 42 | 25 | 24.3 | 18.9 | 1.21 | 1.41 |
| | 40.3 | 29.3 | 42.7 | 29.4 | 24.5 | 19.8 | | |
| | | | | | | | | |
| SA-60N | 50.0 | 40.1 | 50.6 | 34.8 | 28.4 | 12.5 | 1.50 | 3.23 |
| | 52.2 | 41.7 | 49.7 | 36.3 | 26.3 | 9.5 | | |
| | | | | | | | | |
| SA-65N | 54.5 | 44.7 | 55.8 | 41.4 | 24.6 | 8.4 | 1.97 | 4.66 |
| | 55.6 | 45.5 | 56.1 | 42.1 | 21.3 | 9.5 | | |
| | | | | | | | | |
| SA-70N | 55.9 | 45.0 | 56.8 | 42.6 | 26.5 | 7.1 | 1.89 | 6.37 |
| | 57.0 | 45.8 | 56.8 | 42.0 | 21.5 | 6.2 | | |
| | | | | | | | | |
| SA-70S | 55.0 | 45.8 | 55 | 43.2 | 17.7 | 6.6 | 2.75 | 6.39 |
| | 53.4 | 43.7 | 55.9 | 43.1 | 14.9 | 6.9 | | |
| | | | | | | | | |
| IM-1000A | 46.7 | 41.2 | 45.7 | 39.5 | 6.4 | 5.1 | 5.84 | 7.67 |
| | 45.3 | 40.0 | 48.1 | 40.7 | 7.5 | 5.4 | | |

**Table II:**

| Percent Retention (PR) | | | | |
|---|---|---|---|---|
| | RETENTION 1 | | RETENTION 2 | |
| | 0psi ----> 0.3psi | | 0psi ----> 0.7psi | |
| | **PR** | | **PR** | |
| Huggies® PA110607F | 36.5 | 82.7% | 37.1 | 74.4% |
| | | | | |
| Pampers® 1115UG11912002 | 33.8 | 81.4% | 34.8 | 73.9% |
| | | | | |
| J-550 | 47.6 47.0 | 71.2% 73.6% | 48.7 48.7 | 62.2% 64.2% |
| | | | | |
| 10-SHP | 41.1 40.3 | 72.3% 72.7% | 42 42.7 | 59.5% 68.9% |
| | | | | |
| SA-60N | 50.0 52.2 | 80.2% 79.9% | 50.6 49.7 | 68.8% 73.0% |
| | | | | |
| SA-65N | 54.5 55.6 | 82.0% 81.8% | 55.8 56.1 | 74.2% 75.0% |
| | | | | |
| SA-70N | 55.9 57.0 | 80.5% 80.4% | 56.8 56.8 | 75.0% 73.9% |
| | | | | |
| SA-70S | 55.0 53.4 | 83.3% 81.8% | 55 55.9 | 78.5% 77.1% |
| | | | | |
| IM-1000A | 46.7 45.3 | 88.2% 88.3% | 45.7 48.1 | 86.4% 84.6% |

It can be observed that the most notable superabsorbent was the IM-1000A. While the absorbency under load was low its ability to retain the fluid already absorbed was surprisingly high. In Table I the two columns on the right list the RAR (Retention over AUL Ratio). In Table II the percents reported are "percent retention" (PR), i.e. retention capacity divided by the free swell capacity, the retention data come from Table I.

Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application cover the modifications and variations of this invention provided that they come within the scope of the appended claims and their equivalents.

## Claims

1. An absorbent structure comprising a mixture of fibers and superabsorbent particles, wherein the superabsorbent particles are present in the structure in an amount ranging from 2% to 60% on a weight basis, the superabsorbent particles having a residual monomer content less than 300 ppm, an absorbent capacity under low load of at least about 42 g/g and a 0.30 psi percent retention of at least about 80.5%.

2. The absorbent structure of claim 1 wherein the superabsorbent particles have a 0.70 psi percent retention of at least about 74%.

3. The absorbent structure of claim 1 or claim 2 wherein the superabsorbent particles have a 0.30 psi RAR of at least about 1.60.

4. The absorbent structure of any one of claims 1 to 3 wherein the superabsorbent particles have a 0.70 psi RAR of at least about 5.00.

5. An absorbent article having the absorbent structure of any one of claims 1 to 4 wherein the absorbent article is a sanitary napkin, a diaper, an incontinence product or a nursing pad.

6. A sanitary napkin adapted to be worn in the crotch portion of an undergarment, said napkin having a central absorbent zone; said central absorbent zone comprising a fluid-permeable, body-facing cover layer; a fluid impermeable, garment facing barrier layer, and an absorbent system; said central absorbent zone having an absorbent structure comprising a mixture of fibers and superabsorbent particles, wherein the superabsorbent particles are present in the structure in an amount ranging from 2% to 60% on a weight basis, the superabsorbent particles having a residual monomer content less than 300 ppm, an absorbent capacity under low load of at least about 42 g/g and a 0.30 psi Percent Retention of at least about 80.5%.

7. The sanitary napkin of claim 6, wherein said absorbent structure has a thickness from 0.5 mm to 2.5 mm

8. The sanitary napkin of claim 6 or claim 7, wherein said sanitary napkin further comprises a pair of flexible flaps, each flap extending laterally from a longitudinal side of the central absorbent zone.

9. The sanitary napkin of any one of claims 6 to 8, wherein said sanitary napkin further comprises embossed channels.
